Europäisches Patentamt

European Patent Office

Office européen des brevets

(19)

(11) Publication number: **0 222 550**
**A1**

(12) **EUROPEAN PATENT APPLICATION**

(21) Application number: **86308381.2**

(22) Date of filing: **28.10.86**

(51) Int. Cl.⁴: **A61F 5/453**

(30) Priority: **28.10.85 GB 8526531**

(43) Date of publication of application:
**20.05.87 Bulletin 87/21**

(84) Designated Contracting States:
**CH DE ES FR IT LI SE**

(71) Applicant: **Negretti Aviation Limited**
**Edinburgh Place Edinburgh Way**
**Harlow Essex CM20 2DJ(GB)**

(72) Inventor: **Tweedle, John**
**300 Wharf Road Ealand**
**Crowle Scuthorpe, DN17 4JN(GB)**
Inventor: **Mann, Ronald William**
**32 Greenhills**
**Harlow Essex, CM20 3SX(GB)**

(74) Representative: **Sommerville, John Henry**
**SOMMERVILLE & RUSHTON 11 Holywell Hill**
**St. Albans Hertfordshire, AL1 1EZ(GB)**

(54) **Urination facility.**

(57) The facility is of the kind in which a sheath 1 is worn on the penis and defines an aperture 4 at its head end for connection to a collection chamber via a conduit.

There is a problem that the path to the conduit can become blocked when the head of the sheath is not fitting tightly to the penis and the slack material collapses or folds.

To prevent this, the sheath is provided with a number of surface interruptions such as ribs 6, which are spaced around the inner periphery of the sheath and extend, in a spiral, generally longitudinally along the sheath. By this means, if the head of the sheath collapses, open channels are, nevertheless, left open for the passage of urine into the conduit.

FIG.1

## URINATION FACILITY

This invention relates to urination facilities and in particular to a facility of the kind in which a sheath is worn on the penis and defines an aperture at its head end for connection to a bag or other collection container via a conduit to collect urine.

It has been established that a sheath worn on the penis for the purpose of collecting and directing discharging urine can be occluded due to movement of the sheath on the penis and/or movement of clothing. Thus, the sheaths are formed as relatively thin mouldings of rubber latex or similar elastic material and, particularly if there is spare material left at the end of the penis when fitted, there is a likelihood that this spare material will collapse and perhaps fold. Due to the smoothness of the sheath material, when such collapsing and/or folding occurs, the passage through the sheath is cut-off i.e. it occludes, thus preventing the flow of urine into the conduit. This, in turn, can cause a build up in fluid pressure within the sheath, and the urine can then be forced back up through the sheath and leak through the securement arrangement at the base end thereof.

A variety of methods have been used in an attempt to reduce the problem. Thus, mouldings of substantial thickness have been used to reduce the possibility of collapse and folding. However, these are bulky, uncomfor able and expensive. Also, sheaths have been manufactured in a variety of sizes to be a good fit with the minimum of spare material. Furthermore, a variety of methods for securing the sheath so as to avoid spare material being left have been tried.

An object of this invention is to provide a sheath in which occlusion cannot occur even if the sheath moves and is collapsed.

According to this invention, a sheath for a urination facility of the kind described above is formed with elongate surface interruptions, such as ribs, which are spaced from each other and extend longitudinally of the sheath, with at least part of their length at an angle to the longitudinal axis of the sheath, the interruptions being arranged such that, in the event of the sheath collapsing or folding, they act against each other to leave open clear channels for the passage of urine to said conduit.

Preferably, the surface interruptions comprise a number of ribs spaced around the inner circumference of the sheath and which extend longitudinally of the sheath in a spiral whereby to ensure that interlocking of the ribs cannot take place should the sheath collapse or fold.

In order that the invention may be readily understood, one embodiment of a sheath in accordance therewith, and modifications thereof, will now be described with reference to the accompanying drawings, in which:-

Figure 1 is a longitudinal elevation of the sheath,

Figure 2 is a section on the line II-II of Figure 1, and,

Figure 3 is a view similar to Figure 1 showing an alternative securement arrangement.

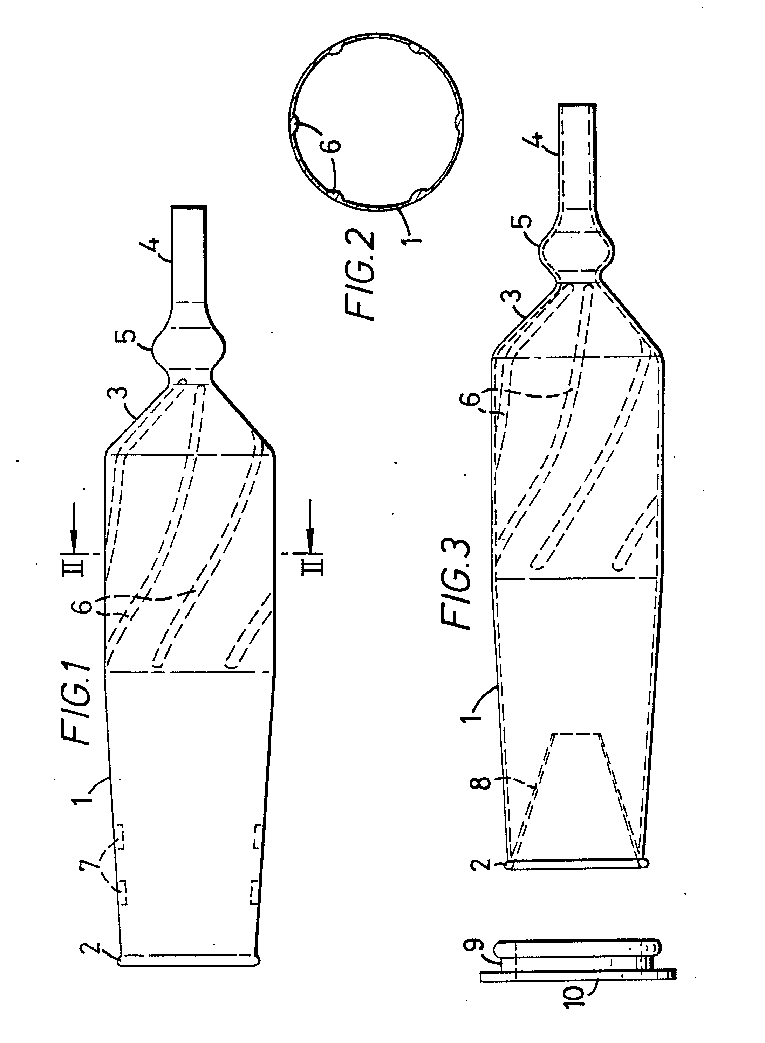

Referring to Figures 1 and 2 the sheath is formed as a moulding from rubber latex and comprises a body portion 1 the inner base end of which is defined by an annular bead 2 of thick material. The outer head end 3 is tapered inwardly to a smaller diameter tube portion 4 having a shaped surge chamber 5 therein. The tube portion is adapted to connect to a conduit for conducting urine to a collecting bag or other appropriate disposal facility in a manner known per se. The thickness of the moulding is preferably around 0.10 to 0.13mm for the body portion 1, which thickens out to 1.0 to 1.2mm for the tube portion 4 and surge chamber 5. These dimensions provide a comfortable sheath for the length of the penis and a sufficiently strong and resilient connection to the conduit.

In accordance with the invention the inner wall surface towards the head of the sheath is provided with projections in the forms of ribs 6 which extend generally longitudinally towards the head end 3. As shown a number of ribs 6 are spaced around the inner circumference of the sheath (Figure 2) and each rib spirals to ensure that interlocking of the ribs cannot take place should the wall of the sheath collapse or fold. However, the ribs could otherwise be straight, or any other suitable form of non-locking protrusion could be used so that, should the wall collapse or fold, the ribs or protrusions will act to ensure that the channels are left for passage for urine to the conduit.

It is proposed that the ribs be approximately 1.0mm thick and 2.0mm or more wide to be effective and to ensure that no appreciable discomfort will be experienced by the user.

The sheath may be secured in position using any of the normally accepeted methods, i.e. adhesives, double-sided adhesive strips 7 (see Figure 1), body belts etc.

An important consideration is to provide a seal at the base end of the sheath. In the case of adhesive strips 7 (see Figure 1) a perfect seal cannot be ensured since leaking can occur past the joints at the ends of the strips. However, in accordance with the invention described and claimed in our co-pending Patent Application (Ref. PA 1988) a membrane seal is proposed which can be moulded separately or, as shown in Figure 3, integrally with the sheath. Referring to Figure 3, the membrane seal 8 is of thin material and is frusto-conical. The length of the seal is sufficient to allow some movement of the penis within the sheath. It will be seen in Figure 3 that the base end 2 of the sheath is intended to be fitted into a groove 9 formed in an annular ring 10 which is appropriately supported. Because the membrane seal 8 permits relative movement between the penis and sheath, it is not so important that the ring 10 can be strapped firmly in position. Instead, it can be supported in an undergarment, e.g. an athletic support, trunks, or the like.

It will be appreciated that with a sheath as described above, because urine flow is not prevented if the sheath collapses, the fit of the sheath on the penis is not so critical, since loose material at the head of the penis can be tolerated. This enables the number of sheath sizes required to be reduced or may even enable a single size sheath to be used.

It will also be appreciated that, apart from the ring 10 and its support, which can be readily washed and reused, the other components are disposable.

The facility as described above is particularly suitable for use by aircrew especially where such crews are carrying out their duties under extreme conditions requiring them to wear sealed flying clothing. However, it will be appreciated that the facility could equally well be used generally for incontinence sufferers.

## Claims

1. A sheath for a urination facility of the kind in which a sheath is worn on the penis which defines an aperture at its head end for connection to a container, via a conduit, to collect urine, wherein the improvement comprises said sheath being formed with elongate surface interruptions such as ribs which are spaced from each other and extend longitudinally of the sheath, with at least part of their length at an angle to the longitudinal axis of the sheath, the interruptions being arranged such that in the event of the sheath collapsing or folding, they act against each other to leave open clear channels for the passage of urine to said conduit.

2. A sheath as claimed in Claim 1, wherein the interruptions comprise a number of ribs spaced around the inner circumference of the sheath and which extend longitudinally of the sheath in a spiral whereby to ensure that interlocking of the ribs cannot take place should the sheath fold or collapse.

3. A sheath as claimed in Claim 1 or 2, wherein a membrane seal is formed internally with the base end of the sheath and wherein an annular ring is provided for strapping to the body around the base of the penis and to which the base end of the sheath is fitted, said membrane being of frusto-conical shape, the smaller end of which fits elastically.on to the penis, and of a length sufficient to allow movement of the penis within the sheath.

FIG.1

FIG.2

FIG.3

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | EP-A-0 108 736 (HANS TOLLIN) <br> * Figures; claims 1,5; page 4, lines 3-5 * | 1 | A 61 F 5/453 |
| Y | | 3 | |
| Y | FR-A-2 083 122 (STILLE WERNER) <br> * The whole document * | 3 | |
| P,X | EP-A-0 185 809 (MANFREDI) <br> * Figures 9-14; page 11, line 10 - page 12, line 6 * | 1 | |
| A | US-A-3 721 243 (HESTERMAN) | | |
| | | | TECHNICAL FIELDS SEARCHED (Int. Cl.4) |
| A | US-A-2 544 201 (WADE) | | A 61 F |

The present search report has been drawn up for all claims

| Place of search | Date of completion of the search | Examiner |
|---|---|---|
| THE HAGUE | 29-01-1987 | STEENBAKKER J. |